# EUROPEAN PATENT APPLICATION

(11) **EP 3 124 473 A1**
(43) Date of publication of application: **01.02.2017**
(21) Application number: 15179388.2
(22) Date of filing: 31.07.2015
(51) Int. Cl.: C07D 231/16, C06B 25/04, C06B 33/10

(54) **BIS(3,4,5-TRINITROPYRAZOLYL)METHANE AND BIS(3,5-DINITRO-4-AMINOPYRAZOLYL)METHANE AS EXPLOSIVES**

(71) Applicant: Ludwig-Maximilians-Universität München, 80539 München (DE)
(72) Inventor: FISCHER, Dennis, 86424 Dinkelscherben (DE); KLAPÖTKE, Thomas, Prof. Dr., 81477 München (DE); STIERSTORFER, Jörg, Dr., 82237 Wörthsee (DE)
(74) Representative: Dr. Gassner & Partner mbB

(57) **Abstract**

The invention relates to bis(3,4,5-trinitropyrazolyl)methane (UMX1), bis(3,5-dinitro-4-aminopyrazolyl)methane (UMX2), an energetic active mass comprising at least one of these compounds, a use of at least one of these compounds as well as methods for synthesizing these compounds.

## Description

The invention relates to bis(3,4,5-trinitropyrazolyl)methane (UMX1 or BTNPM) and its source product bis(3,5-dinitro-4-aminopyrazolyl)methane (UMX2 or BDNAPM), to an energetic active mass comprising these substances, to a use of these substances as well as to methods for synthesizing these substances. The lack of high-performing secondary explosives with both adequate performance and high oxygen content which explosives have a good thermal stability and can be synthesized in a feasible way is known. In explosive formulations a binder is usually used to improve the processing properties of the formulation. The binder in such formulations is usually a material which decreases the oxygen balance significantly. If an oxygen rich explosive is used in the formulation the oxygen balance can be kept at an acceptable level without having "underoxidized" detonation products like methane, ethylene, HCN and so forth. With all the carbon and hydrogen content in the formulation being oxidized to water and carbon monoxide, an optimum performance can be achieved.

Known secondary explosives are hexogen (RDX), octogen (HMX), hexanitrohexaazaisowurtzitane (CL-20) and nitropenta (PETN).

From Yin, P. et al., Chem. Eur. J., 2014, 20, pages 16529 to 16536 N,N'-ethylene-bridged 4,4'-diaminobis(pyrazole) and its derivatives are known. The density and detonation velocity of these substances is not particularly high.

From Claramunt, R. M. et al., Bulletin de la Société Chimique de France, 1983, No. 1-2, pages II-5 to II-10, methylene-1,1'-dipyrazole derivatives having amino or nitro substituents are known.

From DE 38 20 739 A1 metal salts of halogen substituted pyrazoles are known.

From Dalinger, I. L. et al., Synthesis, 2012, 44, pages 2058 to 2064 4-chloro-3,5-dinitropyrazole and the formation of a salt thereof is known.

From WO 96/20146 the use of pyrazole derivatives with hydrophobic groups as nitrification inhibitors is known. One of the derivatives is a substituted bispyrazolylmethane.

The problem to be solved by the present invention is to provide alternative energetic substances, an energetic active mass comprising at least one of these substances, a use of at least one of these substances and methods for synthesizing these substances.

The problem is solved by the subject-matter of claims 1 to 3, 5, 6 and 11. Embodiments of the invention are subject-matter of claims 4, 7 to 10, 12 and 13.

According to the invention bis(3,4,5-trinitropyrazolyl)methane (UMX1, BTNPM) and bis(3,5-dinitro-4-aminopyrazolyl)methane (UMX2, BDNAPM) are provided. Both substances have been found to be high performing explosives. Furthermore, an energetic active mass comprising UMX1, UMX2 or a mixture thereof and a use of UMX1, UMX2 or a mixture thereof are provided according to the invention.

An energetic active mass according to the invention is an active mass that deflagrates or explodes after its ignition. The active mass may be a pyrotechnic active mass. The energetic active mass according to the invention can comprise or consist of a mixture of UMX1 and a powdered metal, such as aluminum or magnesium. The mixing of UMX1 with the powdered metal, in particular aluminum, results in an exceedingly high explosion energy and therewith a high impulse upon detonation. This feature in combination with the high performance of the substance as such is unique. The high impulse upon detonation is particularly useful in ammunition for torpedoes.

The detonation velocity of UMX1 is higher than that of PETN, RDX or HMX. Also the density of UMX1 is higher than that of PETN, RDX or HMX. The calculation of the detonation velocity as well as the energy of explosion was performed by use of the program EXPLO5, Version 6.02 (M. Sućeska, EXPLO5 V6.02 program, Brodarski Institute, Zagreb, Croatia, 2014; M. Sućeska, Calculation of detonation parameters by EXPLO5 computer program, Materials Science Forum, 2004, 465-466, 325-330; M. Sućeska, Calculation of the detonation properties of C-H-N-O explosives, Propellants, Explos., Pyrotech. 1991, 16, 197-202; M. Suceska, Evaluation of detonation energy from EXPLO5 computer code results, Propellants, Explos., Pyrotech. 1999, 24, 280-285; M. L. Hobbs, M. R. Baer, Proc. of the 10th Symp. (International) on Detonation, ONR 33395-12, Boston, MA, July 12-16, 1993, p. 409).

The features of UMX1 and UMX2 compared to PETN, RDX, β-HMX and ε-CL-20 are shown in the following table:

| | **PETN** | **RDX** | **HMX** | **CL-20** | **UMX1** | **UMX2** | **85% w/w UMX1 + 15% w/w Al** |
|---|---|---|---|---|---|---|---|
| Formula | C₅H₈N₄O₁₂ | C₃H₆N₆O₆ | C₄H₈N₈O₈ | C₆H₆N₁₂O₁₂ | C₇H₂N₁₀O₁₂ | C₇H₆N₁₀O₈ | |
| MW [g mol⁻¹] | 316.1 | 222.12 | 296.16 | 438.2 | 418.15 | 358.19 | |
| IS [J]^{a} | 4 | 7.5 | 7 | 4 | 4 | 11 | |
| FS [N]^{b} | 80 | 120 | 112 | 48 | 144 | >360 | |
| ESD-test [J]^{c} | 0.1 | 0.2 | 0.2 | - | 0.1 | >1.5 | |
| *N*[% w/w]^{d} | 17.72 | 37.8 | 37.8 | 38.3 | 33.50 | 39.10 | |
| *Ω*[% w/w]^{e} | -10.12 | -21.61 | -21.61 | -11.0 | -11.48 | -40.20 | -23.1 |
| *T*_{dec}. [°C]^{f} | 150 | 210 | 285 | 195 | 205 | 310 | |
| Density [g cm⁻³]^{g} | 1.778 | 1.80 | 1.905 | 2.038 | 1.934 | 1.802 | 2.02 |
| Δ_{f}*U*° [kJ kg⁻¹]^{h} | -1611 | 415,6 | 353.0 | 982 | 976.8 | 655.8 | |
| -Δ*_{E}U*° [kJ kg⁻¹]ⁱ | 6190 | 5843 | 5794 | 6473 | 6254 | 5052 | 8003 |
| *T*_{E}[K]^{j} | 4306 | 3814 | 3687 | 4654 | 4570 | 3580 | 5527 |
| *p_{CJ}*[kbar]^{k} | 320 | 342 | 389 | 446 | 393 | 295 | 360 |
| *D*[m s⁻¹]^{l} | 8320 | 8838 | 9235 | 9342 | 9300 | 8372 | 8583 |
| Gas vol. [L kg⁻¹] ^{m} | 688 | 786 | 767 | 669 | 704 | 706 | 524 |
| SSRT [mg SiO₂]ⁿ | | 858 | 971 | | 895 | 726 | |

| | | | | | | | |
|---|---|---|---|---|---|---|---|
| ^{a} impact sensitivity (BAM drophammer method, value of a given drop energy if the result of at least one of six tests was positive for that drop energy); ^{b} friction sensitivity (BAM friction tester, value of a given friction force if the result of at least one of six tests was positive for that friction force); ^{c} OZM electrostatic discharge device ; ^{d} nitrogen content; ^{e} oxygen balance; ^{f} decomposition temperature from DSC (measured at a heating rate of 5 °C min⁻¹); ^{g} values calculated for a temperature of 298 K based on X-ray diffraction measurements; ^{h} calculated (CBS-4M) energy of formation; ⁱ energy of explosion; ^{j} explosion temperature; ^{k} detonation pressure; ^{l} detonation velocity; ^{m} assuming only gaseous products; ⁿ dent size measured by filling the dent with powdered SiO₂ and measuring the resulting weight. | | | | | | | |

For the calculation of the energy of formation according to the table the enthalpies (H) and free energies (G) were calculated using the complete basis set (CBS) method of Petersson and coworkers in order to obtain very accurate energies. The CBS models use the known asymptotic convergence of pair natural orbital expressions to extrapolate from calculations using a finite basis set to the estimated complete basis set limit. CBS-4 begins with a HF/3-21 G(d) geometry optimization; the zero point energy is computed at the same level. It then uses a large basis set SCF calculation as a base energy, and a MP2/6-31+G calculation with a CBS extrapolation to correct the energy through second order. A MP4(SDQ)/6-31+(d,p) calculation is used to approximate higher order contributions. For the calculation of the energies of formation according to the table the modified CBS-4M method (M referring to the use of minimal population localization) was applied. The CBS-4M method is a re-parameterized version of the original CBS-4 method and also includes some additional empirical corrections.

DSC measurements at a heating rate of 5 °C/min revealed that UMX1 is thermally stable up to 205 °C. Based on X-ray diffraction measurements the density of the material was calculated to be 1.934 g/cm³ for a temperature of 298 K. This density provides the material with a good energetic performance. The oxygen balance of UMX 1 is -11.48% w/w.

"85% w/w UMX1 + 15% w/w Al" means a mixture of 85% w/w of a powder of UMX1 and 15% w/w of an aluminum powder. Very remarkable is the high energy of explosion of this mixture which energy of explosion is above 8000 kJ/kg. In the "small scale shock reactivity test" (SSRT) UMX2 and UMX1 showed good results. In this test a defined amount of explosive is tamped within a steel housing and is exploded against an aluminum block followed by measuring the volume of the resulting dent by filling this dent with powdered SiO₂ and measuring the resulting weight. The SSRT measures the shock reactivity (explosiveness) of energetic materials. The corresponding result obtained with hexanitrostilbene (HNS) was only 703 mg SiO₂.

UMX1 can be obtained by the oxidation of its precursor UMX2. UMX2 has interesting properties as a thermally stable insensitive explosive. The compound is stable up to 310 °C and has a calculated detonation velocity of 8372 m/s and a detonation pressure of 295 kbar while having low sensitivity values of 11 J towards impact and more than 360 N towards friction. The combination of these values makes the compound unique. A comparison with the common used HNS (hexanitrostilbene) shows that it has a decomposition point which is higher by 10 °C but HNS is the inferior performing explosive.

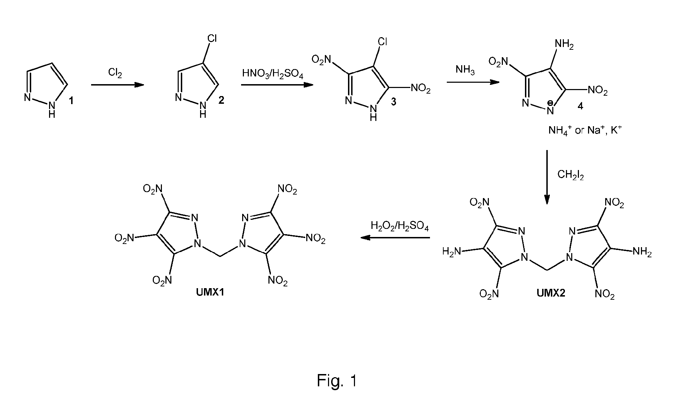

UMX1 can be synthesized in a five step procedure from commercially available pyrazole as shown in Fig. 1. The synthesis of substances 1 to 4 according to Fig. 1 is disclosed in Yin, P. et al., Chem. Eur. J., 2014, 20, pages 16529 to 16536.

UMX2 can be synthesized from 3,5-dinitro-4-aminopyrazolate anions by contacting these anions with a methylene bridge inducing agent in a solution comprising or consisting of a polar solvent. The 3,5-dinitro-4-aminopyrazolate anion can be provided as an ammonium or any alkali or alkaline earth salt, e. g. a sodium salt, of 3,5-dinitro-4-aminopyrazole. The methylene bridge inducing agent may be diiodomethane (CH₂I₂). The solvent may be an aprotic solvent, in particular dimethylformamide. The methylene bridge inducing effect of CH₂I₂ in a solution consisting of dimethylformamide is known in the art, e. g. from Pampaloni, G., et al., Organometallics, 2007, 26(17), pages 4278 to 4286. The 3,5-dinitro-4-aminopyrazolate anions may be contacted with said methylene bridge inducing agent at a temperature of at least 50 °C, in particular at least 60 °C, in particular at least 70 °C, in particular at least 80 °C. The UMX2 can be obtained from the solution by precipitation by reducing polarity in the solution, in particular by addition of water to the solution.

UMX1 can be obtained from UMX2 by oxidation of UMX2. For this purpose H₂O₂, H₂SO₄ or a mixture of H₂O₂ and H₂SO₄ can be used as a means for oxidation and as a solvent for said UMX1 and said UMX2. The UMX1 can be obtained from a solution comprising or consisting of the solvent and UMX1 by precipitation by addition of water to the solution.

Embodiments of the invention:
A) Synthesis of UMX2
   The sodium salt of 3,5-dinitro-4-aminopyrazole can be easily obtained by neutralization of 3,5-dinitro-4-aminopyrazole or by reacting the ammonium salt of it with sodium hydroxide in ethanol or water. In the latter case the formed ammonia is volatile. The sodium salt can be easily recrystallized from water to get a highly pure sample. 4.29 g (22 mmol) sodium 3,5-dinitro-4-aminopyrazolate were suspended in 15 mL DMF and 805 µl (2.68 g, 10 mmol) diiodomethane was added. The mixture was stirred at 90 °C overnight for 16 h and poured on 100 mL of water. A little dilute sodium thiosulfate solution is added to reduce the precipitated iodine (from side reactions) until the suspension gives a nice and clean yellow color. The precipitated product was filtered and washed with water and dried in air to give 3.18 g UMX2 (yield: 89 % mol/mol) as yellow solid.
   The features of the products are as follows:
   **DSC** (5 °C min⁻¹, °C): 310°C (dec.); **IR** (ATR, cm⁻¹): *ṽ* = 3484 (w), 3462 (w), 3368 (w), 3349 (w), 1642 (s), 1579 (w), 1508 (m), 1477 (s), 1445 (m), 1386 (m), 1352 (w), 1300 (s), 1274 (vs), 1234 (m), 1218 (m), 1150 (w), 1103 (w), 1063 (w), 1004 (m), 886 (w), 827 (m), 803 (w), 785 (m), 759 (m), 743 (w), 736 (w); **Raman** (1064 nm, 10 mW, 25 °C, cm⁻¹): *ṽ* = 3352 (3), 3032 (2), 1639 (22), 1568 (8), 1471 (8), 1446 (5), 1407 (7), 1391 (27), 1375 (100), 1352 (39), 1316 (4), 1294 (4), 1274 (6), 1238 (10), 1209 (2), 1151 (2), 1007 (6), 834 (46), 802 (6), 792 (12), 740 (11), 676 (2), 638 (5), 358 (9), 226 (2); **¹H NMR** (400 MHz, DMSO-d₆, 25 °C, ppm): δ=7.29, 7.16; **¹³C NMR{¹H}** (400 MHz, DMSO-*d*₆, 25 °C, ppm) δ=142.1, 131.7, 130.9, 67.2; **m/z (DEI⁺):** 358.2 [M⁺]; **EA** (C₇H₆N₁₀O₈, 258.19) calc.: C 23.47, H 1.69, N 39.10 % w/w; found: C 23.73, H 1.81, N 38.90 % w/w; **BAM drophammer:** 11 J (<100 µm); **friction tester:** > 360 N (<100 µm); ESD > 1 J.
B) Synthesis of UMX1
   1000 mg of UMX2 were dissolved in 5 mL concentrated H₂SO₄ and added dropwise to a mixture of 7.5 mL 50% w/w H₂O₂ and 25 mL H₂SO₄ at 0 °C. The mixture is stirred at this temperature for 3 h and overnight at room temperature. After diluting the mixture with 100 mL icewater the compound was isolated by filtration and washed with water and dried in air giving 1.00 g of UMX1 (yield: 86 % mol/mol).
   The features of the products are as follows:
   **DSC** (5 °C min⁻¹): 205 °C (dec.); **IR** (ATR, cm⁻¹): *ṽ* = 1586 (w), 1566 (w), 1541 (vs), 1471 (w), 1363 (w), 1332 (s), 1300 (m), 1263 (w), 1204 (w), 1084 (w), 1072 (w), 1000 (w), 906 (s), 844 (s), 806 (s), 775 (m), 742 (w), 673 (w), 598 (w); **Raman** (1064 nm, 200 mW, 25 °C, cm⁻¹): *ṽ* = 3007 (11), 1573 (14), 1474 (6), 1453 (12), 1427 (100), 1411 (13), 1368 (35), 1334 (61), 1266 (10), 846 (60), 808 (6), 744 (13), 536 (5), 494 (5), 325 (17), 234 (6); **¹H NMR** (acetone-*d₆*, 25 °C, ppm) δ: 7.84; **¹³C**{**¹H**} **NMR** (acetone-*d₆*, 25 °C, ppm) δ:144.2, 138.8, 123.8, 67.3; **¹⁴N NMR** (acetone-*d₆*, 25 °C, ppm) δ: -29.1, -31.0, -33.5; **m/z (DEI⁺):** 372.1 [(M -NO₂)⁺]; **EA** (C₇H₂N₁₀O₁₂, 418.15): calc.: C 20.11, H 0.48, N 33.50 % w/w; found: C 19.91, H 0.82, N 32.55 % w/w; **BAM drophammer:** 4 J; **friction tester:** 112 N; **ESD:** 0.6 J.

In the above features of UMX2 and UMX1 **EA** means "elementary analysis", wherein "calc." means the calculated and "found" means the actually determined percentage by weight of the respective elements. **m/z (DEI⁺)** means the result obtained by mass spectrometry using **D**esorption **E**lectron **I**onisation. The result is given as mass (m) per charge (z), i. e. the mass (in atomic mass units) of the molecule having a single positive charge.

## Claims

1. Bis(3,4,5-trinitropyrazolyl)methane.

2. Bis(3,5-dinitro-4-aminopyrazolyl)methane.

3. Energetic active mass comprising bis(3,4,5-trinitropyrazolyl)methane or bis(3,5-dinitro-4-aminopyrazolyl)methane or a mixture thereof.

4. Energetic active mass according to claim 3, wherein the energetic active mass comprises or consists of a mixture of bis(3,4,5-trinitropyrazolyl)methane and a powdered metal, in particular aluminum or magnesium.

5. Use of bis(3,4,5-trinitropyrazolyl)methane or bis(3,5-dinitro-4-aminopyrazolyl)methane or a mixture thereof as explosive.

6. Method for synthesizing bis(3,5-dinitro-4-aminopyrazolyl)methane wherein 3,5-dinitro-4-aminopyrazolate anions are contacted with a methylene bridge inducing agent in a solution comprising or consisting of a polar solvent.

7. Method according to claim 6, wherein the methylene bridge inducing agent is diiodomethane.

8. Method according to claim 6 or 7, wherein the solvent is an aprotic solvent, in particular dimethylformamide.

9. Method according to any of claims 6 to 8, wherein the 3,5-dinitro-4-amino-pyrazolate anions are contacted with said methylene bridge inducing agent at a temperature of at least 50 °C, at least 60 °C, at least 70 °C or at least 80 °C.

10. Method according to any of claims 6 to 9, wherein the bis(3,5-dinitro-4-aminopyrazolyl)methane is obtained from the solution by precipitation by reducing polarity in the solution, in particular by addition of water to the solution.

11. Method for synthesizing bis(3,4,5-trinitropyrazolyl)methane wherein bis(3,5-dinitro-4-aminopyrazolyl)methane is oxidized to yield bis(3,4,5-trinitropyrazolyl)methane.

12. Method according to claim 11, wherein H₂O₂, H₂SO₄ or a mixture of H₂O₂ and H₂SO₄ is used as a means for oxidation and as a solvent for said bis(3,5-dinitro-4-aminopyrazolyl)methane and said bis(3,4,5-trinitropyrazolyl)methane.

13. Method according to claims 11 or 12, wherein the bis(3,4,5-trinitropyrazolyl)methane is obtained from a solution comprising or consisting of the solvent and bis(3,4,5-trinitropyrazolyl)methane by precipitation by addition of water to the solution.
